# EUROPEAN PATENT SPECIFICATION

(11) **EP 3 065 568 B1**
(45) Date of publication and mention of the grant of the patent: **25.12.2019**
(21) Application number: 14799699.5
(22) Date of filing: 06.11.2014
(51) Int. Cl.: A23L 33/10, A61K 9/16, C07C 403/24

(54) **PROCESS FOR THE PURIFICATION OF ASTAXANTHIN**
VERFAHREN ZUR REINIGUNG VON ASTAXANTHIN
PROCESSUS DE PURIFICATION D'ASTAXANTHINE

(30) Priority: 07.11.2013 US 201361901136 P; 12.11.2013 CH 18912013
(43) Date of publication of application: 14.09.2016
(73) Proprietor: DSM IP Assets B.V., 6411 TE Heerlen (NL)
(72) Inventor: KARRER, Philippe, CH-4303 Kaiseraugst (CH); WUESTENBERG, Bettina, CH-4303 Kaiseraugst (CH)
(74) Representative: Steck, Melanie
(86) International application number: PCT/EP2014/073955
(87) International publication number: WO 2015/067706

(56) References cited:
- EP-A1- 0 633 258
- WO-A1-01/19383
- WO-A1-2007/128574
- WO-A1-2009/144329
- WO-A1-2010/058235
- WO-A2-2007/072529
- CN-A- 103 044 304
- AMBATI RANGA RAO ET AL: "Stabilization of astaxanthin in edible oils and its use as an antioxidant", JOURNAL OF THE SCIENCE OF FOOD AND AGRICULTURE, vol. 87, no. 6, 30 April 2007 (2007-04-30) , pages 957-965, XP055120279, ISSN: 0022-5142, DOI: 10.1002/jsfa.2766

## Description

The object of the invention is to provide a synthetic food-grade astaxanthin (AXN) which is not known so far. "Food-grade" within the present invention means "suitable for human consumption". Such a food-grade synthetic AXN thus fulfills all regulatory requirements concerning purity and can be used in the commercial production of food and dietary supplements.

AXN can be manufactured by chemical synthesis (see EP-A 908 449, WO 2005/087720, EP-A 733 619), it can be produced by fermentation (see EP-A 543 023) and isolated from natural sources such as shell waste (see JP-A 11-049 972) or algae *Haematococcus pluvialis* (see GB-A 2,301,587), where the isolated esters would have to be cleaved to AXN itself.

AXN isolated from natural sources is already used in products for human consumption: see e.g. US 2009/0297492, where AXN is described as improving the cognitive performance; US 2009/0018210, where AXN is described as promoting fat degradation; EP-A 1 867 327, where AXN is described for preventing a neurodegenerative disease - to name only a few. So far, however, synthetic AXN was not used in products for human consumption.

Synthetic AXN is of standardized quality compared to AXN from natural sources since it is much easier to use a standardized procedure for its chemical synthesis and purification than being dependent from the varying quality of natural AXN resulting by using natural sources which vary also in their composition. Thus, in the context of the present invention the term "AXN" means "synthetic AXN".

Chemical processes for the manufacture of AXN are often carried out in halogenated hydrocarbons such as dichloromethane (see e.g. WO 2011/095571) since AXN and its precursors have a high solubility in these solvents though there were intentions in the past to avoid the use of such solvents (see e.g. EP-A 908 449 and WO 2005/087720). There are also purification methods that use chloroform (see JP-A 07118226). The thus obtained AXN is then often crystallized from lower alkanols since it is hardly soluble in such solvents

There exist also processes for the purification of astaxanthin: In example 11 of WO 2007/072529 the synthesis of astaxanthin is performed in isopropanol. The obtained product is recrystallized in three successive rounds: one with methylene chloride, one with methanol and one with heptane. Hereby all astaxanthin is dissolved. According to WO 2007/128574 (see page 9, line 25) the filter cake of astaxanthin is washed with methanol. According to EP-A 633 258 (see example 2) the astaxanthin crystals have been washed with heptane.

It was an object of the present invention to provide a synthetic AXN which is of reliable quality, easy to obtain also in an industrial scale and suitable for human consumption.

Thus, it was an object of the present invention to provide a food-grade synthetic AXN.

This object is met by the process of the present invention which yields an AXN with extremely low solvent levels, preferably with extremely low levels of dichloromethane, thus rendering this AXN suitable for human consumption. Such an AXN is also an object of the present invention.

Thus, the present invention is especially directed to the preparation of a synthetic AXN with a content of dichloromethane ≤ 250 ppm, preferably with a content of dichloromethane ≤ 200 ppm, more preferably with a content of dichloromethane ≤ 100 ppm, even more preferably with a content of dichloromethane ≤ 50 ppm, ≤ 35 ppm, ≤ 30 ppm, ≤ 25 ppm, ≤ 20 ppm, most preferably with a content of dichloromethane ≤ 10 ppm.

In an especially preferred embodiment of the present invention the synthetic AXN has a content of dichloromethane in the range of between 0 and 100 ppm, preferably in the range of between 10 and 100 ppm.

Preferably the synthetic AXN prepared according to the present invention has a content of methanol ≤ 500 ppm, preferably it has a content of methanol ≤ 350 ppm, more preferably it has a content of methanol ≤ 250 ppm, even more preferably it has a content of methanol ≤ 150 ppm, ≤ 100 ppm, most preferably it has a content of methanol ≤ 50 ppm, ≤ 20 ppm, ≤ 10 ppm, ≤ 5 ppm.

In an especially preferred embodiment of the present invention the synthetic AXN has a content of methanol in the range of between 0 and 50 ppm, preferably in the range of between 0 and 20 ppm, more preferably in the range of 0 to 10 ppm, most preferably in the range of 0 to 5 ppm.

In a further preferred embodiment the synthetic AXN has a content of dichloromethane < 50 ppm and a content of methanol < 100 ppm.

The synthetic AXN prepared according to the present invention may especially be used in dietary supplements. For these purposes it is often provided in the form of an oily suspension or a powdery formulation such as a beadlet, thus protecting the synthetic food-grade AXN from degradation.

Since it is possible with the processes of the present invention to significantly reduce the content of halogenated organic solvents such as dichloromethane, there is no need any more to use a chemical synthesis, where the use of such solvents is avoided.

### Processes

The present invention is directed to a process for the manufacture of synthetic food-grade astaxanthin (starting material: synthetic astaxanthin crystals; PROCESS A) comprising the following steps:
a) Providing synthetic astaxanthin crystals containing dichloromethane in a reactor; and
b) Adding methanol to the synthetic astaxanthin crystals in an amount to obtain a suspension of synthetic astaxanthin in methanol, whereby the amount of synthetic astaxanthin in said suspension is in the range of from 5 to 70 weight-%, preferably in the range of from 20 to 50 weight-%, more preferably in the range of from 30 to 40 weight-%, based on the total weight of the suspension, and closing the reactor; and
c) bringing the suspension obtained in step b) to a temperature in the range of from 80 to 140°C, more preferably to a temperature in the range of from 100 to 120°C, most preferably to a temperature in the range of from 105 to 115°C; and
d) Maintaining the suspension at the temperature as reached in step c); and
e) Bringing the methanolic synthetic astaxanthin suspension to atmospheric pressure by releasing overpressure;
f) Cooling the synthetic astaxanthin suspension to a temperature in the range of 10 to 35°C; and
g) Filtering off the synthetic astaxanthin crystals and drying them; and
whereby during the process the total amount of synthetic astaxanthin is not completely dissolved in the methanol at the same time, and
whereby a synthetic astaxanthin with a content of dichloromethane ≤ 250 ppm is obtained.

Instead of AXN crystals also a solution of AXN in dichloromethane may be used as starting material. Thus, the present invention is also directed to a process for the manufacture of synthetic food-grade astaxanthin (starting material: solution of synthetic astaxanthin in dichloromethane) comprising the following steps (PROCESS B):
i) Providing a solution of synthetic astaxanthin in dichloromethane; and
ii) Adding methanol to the synthetic astaxanthin solution in an amount to obtain a suspension of synthetic astaxanthin, whereby the amount of synthetic astaxanthin in said suspension is in the range of from 5 to 70 weight-%, based on the total weight of the suspension, and closing the reactor; and
iii) bringing the suspension obtained in step ii) to a temperature in the range of from 80 to 140°C; and
iv) Maintaining the suspension at the temperature as reached in step iii); and
v) Bringing the methanolic synthetic astaxanthin suspension to atmospheric pressure by releasing overpressure; and
vi) Cooling the synthetic astaxanthin suspension to a temperature in the range of from 10 to 35°C; and
vii) drying the synthetic astaxanthin suspension obtained in step vi);
whereby during the process the total amount of synthetic astaxanthin is not completely solved in the methanol at the same time, and
whereby a synthetic astaxanthin with a content of dichloromethane ≤ 250 ppm is obtained.

The single steps of the processes of the present invention are now described in detail below.

The processes of the present invention are economic and may be performed in an industrial scale. During the processes according to the present invention the total amount of astaxanthin is not completely dissolved in the methanol. This is in contrast to a crystallization process where the total amount of astaxanthin is completely dissolved upon heating and re-crystallizes when the temperature is decreased again.

Since the steps of Process A and B only differ with respect to the starting material, they are the same and thus, described together.

### Step a)/step i)

As starting material either synthetic AXN crystals (see process A - step a)) or a solution of synthetic AXN in dichloromethane (see process B - step i)) may be used.

Process A: The synthetic AXN crystals are as obtained from any of the chemical syntheses after removing of the solvent, in which the synthesis has been performed as e.g. preferably in dichloromethane. The dichloromethane may have been removed by distillation or by solvent exchange. Preferably the dichloromethane has been removed by a solvent exchange with methanol. In this case the mixture of astaxanthin and dichloromethane is heated up to a temperature of 38 to 40°C at atmospheric pressure and dichloromethane is distilled off. Simultaneously methanol is added so that the volume of the mixture is kept constant. Then solvent (dichloromethane + methanol) are distilled off until the internal temperature has been raised to 64°C (boiling point of methanol) meaning that only methanol is distilled off, but no dichloromethane any more.

These synthetic AXN crystals still contain dichloromethane which cannot be removed by just prolonging the time for drying these synthetic AXN crystals, even if the temperature and/or the vacuum is/are increased. Typical amounts of remaining dichloromethane in the synthetic AXN crystals are 0.2 to 0.3 weight-%, based on the total weight of the synthetic AXN crystals.

Process B: Since the process of the present invention is very effective in removing dichloromethane, it is also possible to use directly a solution of the synthetic AXN in dichloromethane. In this case it is advantageous to first reduce the amount of dichloromethane in the solution by distillation. Preferably this is done whereby simultaneously methanol is added.

Thus, if process B is performed advantageously steps a2) and a3) are also performed.
a2) optionally adding methanol to the synthetic astaxanthin solution of step i);
a3) removing dichloromethane by distillation;
   in case step a2) has been performed removing dichloromethane and methanol by distillation.

### Step b)/step ii)

Preferably step b)/step ii) is carried out in a closed reactor.

Preferably the methanol is added to the synthetic astaxanthin crystals (step b) - Process A) or the solution of synthetic AXN in dichloromethane (step ii) - Process B) in an amount to obtain a suspension of synthetic astaxanthin in methanol, whereby the amount of synthetic astaxanthin in said suspension is in the range of from 20 to 60 weight-%, more preferably in the range of from 30 to 50 weight-%. Preferably this is done under nitrogen.

### Step c)/Step iii)

Preferably the suspension obtained in step b)/step ii) is heated up to a temperature, i.e. brought to a temperature, in the range of from 80 to 140°C, more preferably to a temperature in the range of from 90 to 125°C, even more preferably to a temperature in the range of from 100 to 120°C, most preferably to a temperature in the range of from 105 to 115°C, in a closed reactor. Thereby the pressure increases.

### Step d)/step iv)

The temperature is maintained constant at the wanted value - the same temperature as in step c)/iii).

### Step e)/step v)

Preferably the methanolic synthetic astaxanthin suspension is brought to atmospheric pressure in a time interval of 2 to 5 minutes or the pressure is decreased in a range of 1 to 2.5 bar per minute.

### Step f)/step vi)

Preferably the methanolic synthetic astaxanthin suspension, whereby its content of dichloromethane is ≤ 250 ppm, is cooled to a temperature in the range of from 15-30°C, more preferably to a temperature in the range of from 20 to 25°C.

### Step h)/step h1)

The synthetic astaxanthin crystals are either filtered off (Process A) or not filtered off (Process B) and dried.

The drying of the obtained synthetic AXN crystals is generally carried out at a temperature below 140°C, preferably at a temperature in the range of from 40 to 140°C, and in vacuum. The vacuum is preferably in a range of between 0 and 20 mbara.

In one embodiment of the present invention the drying of the synthetic AXN crystals is carried out at a temperature of 80°C and at 20 mbar. In a further embodiment of the present invention the drying of the synthetic AXN crystals is carried out at a temperature in the range of from 55 to 70°C and at a pressure below 20 mbar.

Especially preferred embodiments of the present invention are those processes, whereby one or more of the preferred conditions given above are realized. The most preferred processes of the present invention are those, whereby all preferred conditions given above are realized.

The invention is now further illustrated in the following non-limiting examples.

### Examples

### Examples 1-3: Processes for obtaining synthetic food-grade astaxanthin

### Example 1

120 g of astaxanthin (97%) containing 2000 mg/kg of dichloromethane are introduced in a 1000 ml steel autoclave under inert conditions with 280 ml of methanol. The autoclave is closed, the mixture agitated at 350 rpm (rotations per minute) and heated at 105°C for 6 hours. After that a flash is performed by opening the outlet valve bringing the internal pressure to normal pressure in 10 minutes. After that the mixture is cooled to 25°C and diluted with 190 ml of methanol. The crystals are filtered off, washed with 150 ml of methanol and dried in a drying oven for 16 hours at 80°C under 20 mbara. 118.6 g of dry astaxanthin are obtained containing 61 mg/kg (61 ppm) of dichloromethane and 40 mg/kg (40 ppm) of methanol.

### Example 2

120 g of astaxanthin (97%) containing 2000 mg/kg of dichloromethane are introduced in a 1000 ml steel autoclave under inert conditions with 280 ml of methanol. The autoclave is closed, the mixture agitated at 350 rpm and heated at 105°C for 15 minutes. After that a flash is performed by opening the outlet valve bringing the internal pressure to normal pressure in 10 minutes. After that the mixture is cooled to 25°C and diluted with 200 ml of methanol. The crystals are filtered off, washed with 150 ml of methanol and dried in a drying oven for 16 hours at 80°C under 20 mbara. 118.0 g of dry astaxanthin are obtained containing 240 mg/kg of dichloromethane and 45 mg/kg of methanol.

### Example 3

Example 1 can be repeated, whereby the mixture is heated at 110°C for 6 hours.

In principle is it possible to perform example 1, 2 or 3 as described above also at any other temperature in the range of from 80 to 140°C instead of 105°C and 110°C, respectively. Such other temperatures are e.g. 80°C, 85°C, 90°C, 95°C, 100°C, 115°C, 120°C, 125°C, 130°C, 135°C, 140°C and any other temperature in between. Example 1, 2 and 3 may especially be repeated at a temperature of 115°C.

## Claims

1. A process for the manufacture of synthetic food-grade astaxanthin with a content of dichloromethane ≤ 250 ppm comprising the following steps:
a) Providing synthetic astaxanthin crystals containing dichloromethane in a reactor; and
b) Adding methanol to the synthetic astaxanthin crystals in an amount to obtain a suspension of synthetic astaxanthin in methanol, whereby the amount of synthetic astaxanthin in said suspension is in the range of from 5 to 70 weight-%, based on the total weight of the suspension, and closing the reactor; and
c) bringing the suspension obtained in step b) to a temperature in the range of from 80 to 140°C; and
d) Maintaining the suspension at the temperature as reached in step c); and
e) Bringing the methanolic synthetic astaxanthin suspension to atmospheric pressure by releasing overpressure; and
f) Cooling the synthetic astaxanthin suspension to a temperature in the range of 10 to 35°C; and
g) Filtering off the synthetic astaxanthin crystals and drying them;
whereby during the process the total amount of synthetic astaxanthin is not completely solved in the methanol at the same time, and
whereby a synthetic astaxanthin with a content of dichloromethane ≤ 250 ppm is obtained.

2. The process according to claim 1, wherein in step b) the amount of synthetic astaxanthin in said suspension is in the range of from 20 to 50 weight-%, preferably in the range of from 30 to 40 weight-%, based on the total weight of the suspension.

3. The process according to claim 1 and/or claim 2, wherein in step c) the suspension is brought to a temperature in the range of from 100 to 120°C, preferably to a temperature in the range of from 105 to 115°C.

4. A process for the manufacture of synthetic food-grade astaxanthin with a content of dichloromethane ≤ 250 ppm by purification of a solution of synthetic astaxanthin in dichloromethane comprising the following steps:
i) Providing a solution of synthetic astaxanthin in dichloromethane; and
ii) Adding methanol to the synthetic astaxanthin solution in an amount to obtain a suspension of synthetic astaxanthin, whereby the amount of synthetic astaxanthin in said suspension is in the range of from 5 to 70 weight-%, and closing the reactor; and
iii) bringing the suspension obtained in step ii) to a temperature in the range of from 80 to 140°C; and
iv) Maintaining the suspension at the temperature as reached in step iii); and
v) Bringing the methanolic synthetic astaxanthin suspension to atmospheric pressure by releasing overpressure; and
vi) Cooling the synthetic astaxanthin suspension to a temperature in the range of from 10 to 35°C; and
vii) drying the synthetic astaxanthin suspension obtained in step vi);
whereby during the process the total amount of synthetic astaxanthin is not completely solved in the methanol at the same time, and
whereby a synthetic food-grade astaxanthin with a content of dichloromethane ≤ 250 ppm is obtained.

5. The process according to claim 4, wherein in step ii) the amount of synthetic astaxanthin in said suspension is in the range of from 20 to 50 weight-%, preferably in the range of from 30 to 40 weight-%, based on the total weight of the suspension.

6. The process according to claim 4 and/or claim 5, wherein in step iii) the suspension is brought to a temperature in the range of from 100 to 120°C, preferably to a temperature in the range of from 105 to 115°C.

## Patentansprüche

1. Verfahren zur Herstellung von synthetischem Astaxanthin in Lebensmittelqualität mit einem Dichlormethan-Gehalt ≤ 250 ppm, das folgende Schritte umfasst:
a) Bereitstellen von Kristallen von synthetischem Astaxanthin, die Dichlormethan enthalten, in einem Reaktor; und
b) Zugeben von Methanol zu den Kristallen von synthetischem Astaxanthin in einer Menge zum Erhalt einer Suspension von synthetischem Astaxanthin in Methanol, wobei die Menge an synthetischem Astaxanthin in der Suspension im Bereich von 5 bis 70 Gew.-%, bezogen auf das Gesamtgewicht der Suspension, liegt, und Schließen des Reaktors; und
c) Bringen der in Schritt b) erhaltenen Suspension auf eine Temperatur im Bereich von 80 bis 140°C; und
d) Halten der Suspension bei der in Schritt c) erreichten Temperatur; und
e) Bringen der methanolischen Suspension von synthetischem Astaxanthin auf Normaldruck durch Ablassen von Überdruck; und
f) Abkühlen der Suspension von synthetischem Astaxanthin auf eine Temperatur im Bereich von 10 bis 35°C; und
g) Abfiltrieren der Kristalle von synthetischem Astaxanthin und Trocknen der Kristalle;
wodurch während des Verfahrens die Gesamtmenge von sythetischem Astaxanthin nicht zugleich vollständig in dem Methanol gelöst wird, und
wodurch ein synthetisches Astaxanthin mit einem Dichlormethan-Gehalt ≤ 250 ppm erhalten wird.

2. Verfahren nach Anspruch 1, wobei in Schritt b) die Menge von synthetischem Astaxanthin in der Suspension im Bereich von 20 bis 50 Gew.-% liegt, vorzugsweise im Bereich von 30 bis 40 Gew.-%, bezogen auf das Gesamtgewicht der Suspension.

3. Verfahren nach Anspruch 1 und/oder Anspruch 2, wobei in Schritt c) die Suspension auf eine Temperatur im Bereich von 100 bis 120°C und vorzugsweise auf eine Temperatur im Bereich von 105 bis 115°C gebracht wird.

4. Verfahren zur Herstellung von synthetischem Astaxanthin in Lebensmittelqualität mit einem Dichlormethan-Gehalt ≤ 250 ppm durch Reinigung einer Lösung von synthetischem Astaxanthin in Dichlormethan, das folgende Schritte umfasst:
i) Bereitstellen einer Lösung von synthetischem Astaxanthin in Dichlormethan; und
ii) Zugeben von Methanol zu der Lösung von synthetischem Astaxanthin in einer Menge zum Erhalt einer Suspension von synthetischem Astaxanthin, wobei die Menge von synthetischem Astaxanthin in der Suspension im Bereich von 5 bis 70 Gew.-% liegt, und Schließen des Reaktors; und
iii) Bringen der in Schritt ii) erhaltenen Suspension auf eine Temperatur im Bereich von 80 bis 140°C; und
iv) Halten der Suspension bei der in Schritt iii) erreichten Temperatur; und
v) Bringen der methanolischen Suspension von synthetischem Astaxanthin auf Normaldruck durch Ablassen von Überdruck; und
vi) Abkühlen der Suspension von synthetischem Astaxanthin auf eine Temperatur im Bereich von 10 bis 35°C; und
vii) Trocknen der in Schritt vi) erhaltenen Suspension von synthetischem Astaxanthin;
wodurch während des Verfahrens die Gesamtmenge von sythetischem Astaxanthin nicht zugleich vollständig in dem Methanol gelöst wird, und
wodurch ein synthetisches Astaxanthin in Lebensmittelqualität mit einem Dichlormethan-Gehalt ≤ 250 ppm erhalten wird.

5. Verfahren nach Anspruch 4, wobei in Schritt ii) die Menge von synthetischem Astaxanthin in der Suspension im Bereich von 20 bis 50 Gew.-% liegt, vorzugsweise im Bereich von 30 bis 40 Gew.-%, bezogen auf das Gesamtgewicht der Suspension.

6. Verfahren nach Anspruch 4 und/oder Anspruch 5, wobei in Schritt iii) die Suspension auf eine Temperatur im Bereich von 100 bis 120°C und vorzugsweise auf eine Temperatur im Bereich von 105 bis 115°C gebracht wird.

## Revendications

1. Procédé pour la préparation d'astaxanthine synthétique de qualité alimentaire dont la teneur en dichlorométhane est ≤ 250 ppm comprenant les étapes suivantes :
a) la mise à disposition de cristaux d'astaxanthine synthétique contenant du dichlorométhane dans un réacteur ; et
b) l'ajout de méthanol aux cristaux d'astaxanthine synthétique en une quantité pour obtenir une suspension d'astaxanthine synthétique dans du méthanol, la quantité d'astaxanthine synthétique dans ladite suspension se situant dans la plage de 5 jusqu'à 70% en poids, sur la base du poids total de la suspension, et la fermeture du réacteur ; et
c) le fait d'amener la suspension obtenue dans l'étape b) à une température dans la plage de 80 jusqu'à 140°C; et
d) le maintien de la suspension à la température telle qu'obtenue dans l'étape c) ; et
e) le fait d'amener la suspension méthanolique d'astaxanthine synthétique à la pression atmosphérique par le relâchement de la surpression ; et
f) le refroidissement de la suspension d'astaxanthine synthétique à une température dans la plage de 10 jusqu'à 35°C; et
g) la filtration des cristaux d'astaxanthine synthétique et le séchage de ceux-ci;
par lequel pendant le procédé la quantité totale d'astaxanthine synthétique n'est pas entièrement dissoute dans le méthanol au même moment, et
par lequel une astaxanthine synthétique dont la teneur en dichlorométhane est ≤ 250 ppm est obtenue.

2. Procédé selon la revendication 1, dans lequel dans l'étape b) la quantité d'astaxanthine synthétique dans ladite suspension se situe dans la plage de 20 jusqu'à 50% en poids, préférablement dans la plage de 30 jusqu'à 40% en poids, sur la base du poids total de la suspension.

3. Procédé selon la revendication 1 et/ou la revendication 2, dans lequel dans l'étape c) la suspension est amenée à une température dans la plage de 100 jusqu'à 120°C, préférablement à une température dans la plage de 105 jusqu'à 115°C.

4. Procédé pour la préparation d'astaxanthine synthétique de qualité alimentaire dont la teneur en dichlorométhane est ≤ 250 ppm par la purification d'une solution d'astaxanthine synthétique dans le dichlorométhane comprenant les étapes suivantes :
i) la mise à disposition d'une solution d'astaxanthine synthétique dans du dichlorométhane ; et
ii) l'ajout de méthanol à la solution d'astaxanthine synthétique en une quantité pour obtenir une suspension d'astaxanthine synthétique, la quantité d'astaxanthine synthétique dans ladite suspension se situant dans la plage de 5 jusqu'à 70% en poids, et la fermeture du réacteur ; et
iii) le fait d'amener la suspension obtenue dans l'étape ii) à une température dans la plage de 80 jusqu'à 140°C; et
iv) le maintien de la suspension à la température telle qu'obtenue dans l'étape iii) ; et
v) le fait d'amener la suspension méthanolique d'astaxanthine synthétique à la pression atmosphérique par le relâchement de la surpression ; et
vi) le refroidissement de la suspension d'astaxanthine synthétique à une température dans la plage de 10 jusqu'à 35°C; et
vii) le séchage de la suspension d'astaxanthine synthétique obtenue dans l'étape vi) ;
par lequel pendant le procédé la quantité totale d'astaxanthine synthétique n'est pas entièrement dissoute dans le méthanol au même moment, et
par lequel une astaxanthine synthétique de qualité alimentaire dont la teneur en dichlorométhane est ≤ 250 ppm est obtenue.

5. Procédé selon la revendication 4, dans lequel dans l'étape ii) la quantité d'astaxanthine synthétique dans ladite suspension se situe dans la plage de 20 jusqu'à 50% en poids, préférablement dans la plage de 30 jusqu'à 40% en poids, sur la base du poids total de la suspension.

6. Procédé selon la revendication 4 et/ou la revendication 5, dans lequel dans l'étape iii) la suspension est amenée à une température dans la plage de 100 jusqu'à 120°C, préférablement à une température dans la plage de 105 jusqu'à 115°C.
